(19) Europäisches Patentamt
European Patent Office
Office européen des brevets

(11) Publication number: **0 042 228 A2**

# (12) EUROPEAN PATENT APPLICATION

(21) Application number: **81302379.3**

(22) Date of filing: **29.05.81**

(51) Int. Cl.³: **A 01 N 47/12**, C 07 C 125/065

(30) Priority: **17.06.80 GB 8019734**
**05.05.81 GB 8113666**

(43) Date of publication of application: **23.12.81**
**Bulletin 81/51**

(84) Designated Contracting States: **CH DE FR GB IT LI SE**

(71) Applicant: **IMPERIAL CHEMICAL INDUSTRIES LIMITED**
**Imperial Chemical House Millbank, London SW1P 3JF (GB)**

(72) Inventor: **Baker, Raymond, 10 Woodview CLose Bassett, Southampton S02 3PZ Hants (GB)**
Inventor: **Green, Anthony St.John, 9, North View Amen Corner, Binfield Berkshire (GB)**
Inventor: **Jutsum, Alan Richard, 9, Leney Close, Wokingham Berkshire (GB)**
Inventor: **Turnbull, Michael Drysdale, 19, Sombarton Gardens Lower Earley, Reading Berkshire (GB)**

(74) Representative: **Fawcett, Richard Fennelly et al, Imperial Chemical Industries Limited Legal Department: Patents Thames House North Millbank, London SW1P 4QG (GB)**

## (54) Method of protecting plants from insect pests.

(57) The use of insect oviposition inhibitors or stimulators of carbamates of formula (I) or (II)

$$CH_3(CH_2)_kCH{=}CH[(CH_2)_mCH{=}CH]_x(CH_2)_nOCONR^1R^2 \quad (I)$$

$$CH_3(CH_2)_pOCONR^1R^2 \quad (II)$$

wherein k ist 0 to 3, m is 0 to 2, n is 5 to 8, x is 0 or 1, p is 11 to 18 and $R^1$ and $R^2$ is hydrogen or alkyl.

EP 0 042 228 A2

31382\EP

0042228

# METHOD OF PROTECTING PLANTS FROM INSECT PESTS .

This invention relates to a method of protecting crops from insect pests using carbamate compounds, to certain of the carbamate compounds themselves, to a process for preparing them and to agricultural compositions containing them.

One possible way of protecting crops from insect pests is to apply to the crops or to the locus of the crop a chemical compound which has the property of attracting or repelling that insect pest. Compounds capable of doing this are known to occur in nature, usually in plants and insects.

Most of these compounds influence the behaviour of the male insect when the compound is released by the female of that particular insect species. These compounds are known as pheromones and, in addition to naturally occurring pheromones, a number of synthetic pheromones have been prepared.

However, there are some compounds known in nature which influence the behaviour of female insects for example by discouraging or encouraging the female insect to lay eggs. Such compounds can be referred to as oviposition inhibitors or stimulators.

We have now found a group of synthetic compounds which have oviposition inhibiting or stimulating properties.

The invention provides a method of protecting a crop from insect pests, the method comprising applying to the crop, to the locus of the crop of to a locus remote from the crop, a compound of general formula (I) or (II)

$$CH_3(CH_2)_kCH{=}CH[(CH_2)_mCH{=}CH]_x(CH_2)_nOCONR^1R^2 \quad (I)$$

$$CH_3(CH_2)_pOCONR^1R^2 \quad (II)$$

wherein k is 0 or an integer of 1 to 3, m is 0, 1 or 2, n is an integer of 5 to 8, x is 0 or 1, p is an integer of 11 to 18, and each of $R^1$ and $R^2$, which may be the same or different, is hydrogen or $C_{1-4}$ alkyl.

The compounds of general formula (I) form geometrical isomers. The invention includes within its scope such isomers each of which can be isolated from mixtures thereof by methods known in the art. The double bond(s) in the compounds of general formula (I) is (are) generally in the Z-configuration but in some cases a double bond is in the E-configuration.

When $R^1$ and/or $R^2$ is alkyl, it is suitably methyl, but alternatively it can be ethyl, propyl (n- or i- propyl) or butyl (n-, sec-, t- or i- butyl). Preferably one of $R^1$ and $R^2$ is hydrogen and the other is methyl.

Examples of the compounds of general formulae (I) and (II) are given in Tables II and I, respectively.

Table I

| Compound | p | $R^1$ | $R^2$ | Melting (or Boiling) Point (°C) |
|---|---|---|---|---|
| 1 | 11 | H | Me | 49-50° |
| 2 | 12 | H | Me | 59-60° |
| 3 | 13 | H | Me | 61-62° |
| 4 | 14 | H | Me | 66-67° |
| 5 | 15 | H | Me | 70-71° |
| 6 | 13 | Me | Me | (68-69°/0.2mmHg) |

Table II

| Compound | k | x | m | n | $R^1$ | $R^2$ | Melting (or Boiling) Point (°C) |
|---|---|---|---|---|---|---|---|
| 7 | 1 | 0 | 0 | 8 | H | Me | 38-40° |
| 8* | 3 | 0 | 0 | 8 | H | Me | 18-20° |
| 9* | 3 | 0 | 0 | 8 | H | Me | 33-34° |
| 10 | 3 | 0 | 0 | 8 | Me | Me | (71-72/0.2 mm Hg) |
| 11 | 3 | 0 | 0 | 6 | H | Me | (150°/0.1 mm Hg) |
| 12ˣ | 3 | 1 | 2 | 6 | H | Me | (165°/0.03 mm Hg) |

* Compound 8 is in the Z-form and Compound 9 is in the E-form. ˣCompound 12 is a mixture of 50% N-methyl Z,Z-hexadeca-9,12-dien-1-yl carbamate and 50% of its Z, E-isomer.

The compounds of general formula (I) and most of the compounds of general formula (II) are novel compounds and as such form part of the invention. The invention provides a compound of general formula (I) or (II)

$$CH_3(CH_2)_kCH{=}CH[(CH_2)_mCH{=}CH]_x(CH_2)_nOCONR^1R^2 \qquad (I)$$

$$CH_3(CH_2)_pOCONR^1R^2 \qquad (II)$$

wherein k is 0 or an integer of 1 to 3, m is 0, 1 or 2, n is an integer of 5 to 8, x is 0 or 1, p is an integer of 11 to 18, and each of $R^1$ and $R^2$, which may be the same or different, is hydrogen or $C_{1-4}$ alkyl, subject to the proviso that when $R^1$ is hydrogen and $R^2$ is methyl, p is other than 11, 12 or 14.

The compounds of general formula (I) or (II) can be prepared reacting an alcohol of general formula (IIIA) or (IIIB).

$CH_3(CH_2)_kCH{=}CH[(CH_2)_mCH{=}CH]_x(CH_2)_nOH$ (IIIA)

$CH_3(CH_2)_pOH$ (IIIB)

wherein k, m, x, n and p are as defined above, with a compound of general formula (IVA) or (IVB)

$R^1N{=}C{=}O$ (IVA)

$R^1R^2NCOCl$ (IVB)

wherein $R^1$ and $R^2$ are as defined above except that they cannot both be hydrogen, and k, m, x and n are as defined above.

Alternatively, the compounds of general formula (I) or (II) can be prepared by reacting an alcohol of general formula (IIIA) or (IIIB) with phosgene to give a compound of general formula (VA) or (VB)

$CH_3(CH_2)_kCH{=}CH[(CH_2)_mCH{=}CH]_x(CH_2)_nOCOCl$ (VA)

$CH_3(CH_2)_pOH$ (VB)

wherein k, m, x, n and p are as defined above, which is then reacted with a compound of general formula $R^1R^2NH$ wherein each of $R^1$ and $R^2$, which may be the same or different, is hydrogen or $C_{1-4}$ alkyl, or a salt thereof.

Some at least of the alcohol starting material are known compounds. They can be prepared by methods known in the art.

Suitable insects for treatment with the compounds of general formula (I) or (II) are Lepidoptera sp. especially Choristoneura sp. (eg C.fumiferana and C. virgifera), rice stem borers (eg Chilo suppressalis), Pectinophora gossypiella, Heliothis sp. (e.g. H. zea, H.virescens and H. armigera) and Spodopteran sp. (e.g. S.littoralis and S. exigua).

The crops which can be protected from insect pests using the method of the invention are, for example, trees (eg spruce), cereals (eg rice and maize), cotton and tobacco.

The amount of the compounds applied to the crops is suitably 1 to 1000, preferably 5 to 100, g. per hectare.

Most of the compounds of general formula (I) and (II) are oviposition inhibitors. These would normally be used in the method of the invention by applying the compound to the crop which it is desired to protect; the female insects would then be inhibited from laying eggs on the crop. However, some of the compounds may be oviposition stimulators; such compounds could be used either by applying them to a locus remote from the crop which it is desired to protect (and then the female insects would lay their eggs away from the crop) or by applying them to a limited area of the crop which is treated with a conventional insect control means (for example an insecticide).

The compounds may be used as such for protecting crops from insect pests, but are more conveniently formulated into compositions for such usage. The invention thus provides also an agricultural composition comprising a novel compound of general formula (I) or (II) as hereinbefore defined, and a carrier or diluent.

The compounds can be applied in a number of ways, for example they can be applied, in a formulated or unformulated state, to the foliage of a crop, for example they can be sprayed on, dusted on or applied as a cream or paste formulation, or they can be applied as a vapour. Application can be to any part of the crop, for example to the foliage, stems or branches.

The term "crop" as used herein includes seedlings, bushes and trees.

The compounds are preferably used for agricultural and horticultural purposes in the form of a composition.

The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be reformed granules suitable for application to the soil without further treatment. These granules can be made either by impregnating pellets of filler with the active ingredient or by pelleting a mixture of the active ingredient and powdered filler. Compositions for dressing seed,for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g.2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

It is preferred, however, that the compounds are used in a micro-encapsulated form.

Microencapsulation techniques are known in the art. They can be made by interfacial polycondensation of suitable monomer(s), for example a diacid chloride and diamine can be polycondensed to give a polyamide. The microcapsules can contain, in addition to the active ingredient and carriers or diluents, ultraviolet light absorbing agents and antioxidants.

The compounds can be employed in other slow release forms e.g. contained in hollow fibres or plastic laminate strips.

By including suitable additives, for example additives for improving the distribution, adhesive power and resistance to rain on treated surfaces, the different compositions can be better adapted for various utilities.

The compositions may also be in the form of liquid preparations for use as dips or sprays which are generally aqueous dispersions or emulsions containing the active ingredient in the presence of one or surfactants e.g. wetting agent(s), dispersing agent(s) emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds

(for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol.

Other non-ionic agents are the partial esters derived from long fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragancanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). The concentrates suitably contain a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having insecticidal, plant growth regulating, herbicidal or fungicidal activity.

The invention is illustrated by the following Examples; the temperatures are given in degrees Centigrade.

0042228

EXAMPLE 1

N-methyl pentadecan-1-yl carbamate (Compound 4)

Pentadecanol (228.5g) and triethylamine (10ml) were dissolved in petroleum ether (2 litres; bp 60-80°). Methyl isocyanate (87.2g) was added and the mixture stirred at about 20° for 16 hours. A thick precipitate formed which was filtered off and dried in air to give a white solid (270g) which was recrystallised from n-hexane to give the title compound (206g), mp 66-67°.

EXAMPLE 2

N-methyl Z-tetradec-9-en-1-yl Carbamate (Compound 8)

Z-tetradec-9-en-1-ol(4.159g) in toluene (13ml) and methyl isocyanate (1.8ml). Triethylamine (3ml) was added and the mixture stirred at about 50°C for 8 hours and then left overnight for the reaction to reach completion. The toluene was then evaporated from the mixture and the residue was then taken up in methylene chloride, washed with dilute hydrochloric acid and water and dried (magnesium sulphate). The solvent was removed on a rotary evaporator and the crude product, a yellow oil, was distilled under high vacuum to give the title compound (3.797g), boiling point 175°C/0.2mmHg.

EXAMPLE 3

The test compound (5mg) in acetone was applied to filter paper presented in a choice test versus filter paper treated with acetone alone to adult H.virescens insects in a cage (volume 2700 $cm^3$) with greaseproof paper walls and roof. The papers were left overnight and the number of eggs on each filter paper counted. All the eggs hatched successfully. The results are shown in Table III.

Table III

| Compound | Total number of eggs laid on treated plants | Total number of eggs laid on filter paper treated with acetone alone |
|---|---|---|
| 4 | 72 | 130 |
| | 0 | 40 |
| | 32 | 98 |
| 8 | 1 | 35 |
| | 104 | 212 |
| | 0 | 85 |
| | 23 | 38 |
| 9 | 20 | 10 |
| | 29 | 13 |
| | 64 | 25 |
| 11 | | |
| | 85 | 42 |
| | 178 | 32 |
| Controls* | | |
| | 84 | 76 |
| | 103 | 109 |
| | 50 | 46 |
| | 159 | 165 |
| | 166 | 150 |

* A number of controls were used. In each case, just acetone was applied to the filter papers.

EXAMPLE 4

Four potted young cotton plants (height about 30 cms) were sprayed to run off at 5 p.s.i. with an aqueous solution of the test compound. The aqueous solution was prepared by dissolving the test compound in a acetone/ water (1:9) mixture containing 0.01% of Lissapol NX as surfactant. Four similar plants were sprayed under similar conditions with a blank formulation. When dry, the plants were placed in a box-like cage (110 x 55 x 40 cm) having a back front and top of a clear light-transmitting rigid plastic sheet and ends of metal gauze. The floor of the cage, which was also made of metal gauze, was apertured to receive and support the pots in two groups of four, each group being situated in a line parallel to, and adjacent a gauze end. The cage was situated in a darkened room at a constant temperature of 26°. 24 Hours prior to placing the pots in the cage, 20 male and 20 female young adult Heliothis virescens insects were introduced into the cage. The number of eggs laid was recorded 16 hours into the test. The results of the experiment are given in Table ~~III~~ IV.

Table IV

| Compound | Weight(mg) applied to each plant | Total number of eggs laid on treated plants | Total number of eggs laid on plants treated with blank formulation |
|---|---|---|---|
| 8 ~~5~~ | 2.5 | 0 | 45 |
| Control* | - | 221 | 236 |

* The blank formulation was sprayed on both groups of plants to provide this control.

EXAMPLE 5

The experiment of Example 4 was repeated execpt that the insects were introduced into the cage 48 (and not 24) hours prior to placing the pots in the cage.

0042228

The results are given in Table V

Table V

| Compound | Weight(mg) applied to each plant | Total number of eggs laid on treated plants | Total number of eggs laid on plants treated with blank formulation |
|---|---|---|---|
| 4 | 0.625<br>0.625 | 18<br>0 | 57<br>58 |
| control | - | 221 | 236 |

RFF/SPEC140/bgg
14 May 81

1. A method of protecting a crop from insect pests, characterised by applying to the crop, to the locus of the crop or to a locus remote from the crop, a compound of general formula (I) or (II)

$$CH_3(CH_2)_kCH{=}CH[(CH_2)_mCH{=}CH]_x(CH_2)_nOCONR^1R^2 \quad \text{(I)}$$

$$CH_3(CH_2)_pOCONR^1R^2 \quad \text{(II)}$$

wherein k is 0 or an integer of 1 to 3, m is 0, 1 or 2, n is an integer of 5 to 8, x is 0 or 1, p is an integer of 11 to 18, and each of $R^1$ and $R^2$, which may be the same or different, is hydrogen or $C_{1-4}$ alkyl.

2. A method according to claim 1 characterised in that the compound of general formula (I) is N-methyl Z-tetradec-9-en-1-yl carbamate.

3. A method according to claim 1 characterised in that the compound of general formula (II) is N-methyl pentadecan-1-yl carbamate.

4. A compound of general formula (I) or (II)

$$CH_3(CH_2)_kCH{=}CH[(CH_2)_mCH{=}CH]_x(CH_2)_nOCONR^1R^2 \quad \text{(I)}$$

$$CH_3(CH_2)_pOCONR^1R^2 \quad \text{(II)}$$

wherein k is 0 or an integer of 1 to 3, m is 0, 1 or 2, n is an integer of 5 to 8, x is 0 or 1, p is an integer of 11 to 18, and each of $R^1$ and $R^2$, which may be the same or different, is hydrogen or $C_{1-4}$ alkyl, subject to the proviso that when $R^1$ is hydrogen and $R^2$ is methyl, p is other than 11, 12 or 14.

5. N-methyl Z-tetradec-9-en-1-yl carbamate.

6. N-methyl pentadecan-1-yl carbamate.

7. A process for preparing a compound according to any one of claims 4 to 6, characterised by comprising (a) reacting an alcohol of general formula (IIIA) or (IIIB)

$$CH_3(CH_2)_kCH{=}CH[(CH_2)_mCH{=}CH]_x(CH_2)_nOH \qquad (IIIA)$$

$$CH_3(CH_2)_pOH \qquad (IIIB)$$

wherein k, m, x, n and p are as defined in claim 4, with a compound of general formula (IVA) or (IVB)

$$R^1N{=}C{=}O \qquad (IVA)$$

$$R^1R^2NCOCl \qquad (IVB)$$

wherein $R^1$ and $R^2$ are as defined in claim 4 except that they cannot both be hydrogen; or (b) reacting a compound of general formula (VA) or (VB)

$$CH_3(CH_2)_kCH{=}CH[(CH_2)_mCH{=}CH]_x(CH_2)_nOCOCl \qquad (VA)$$

$$CH_3(CH_2)_pOCOCl \qquad (VB)$$

wherein k, m, x, n and p are as defined in claim 4, with a compound of general formula $R^1R^2NH$ wherein each of $R^1$ and $R^2$, which may be the same or different, is hydrogen or $C_{1-4}$ alkyl, or a salt thereof.

8. A composition suitable for protecting a crop from insect pests, characterised by comprising a compound according to any one of claims 4 to 6, and a carrier or diluent.